# EUROPÄISCHE PATENTSCHRIFT

(11) **EP 0 820 760 B1**
(45) Veröffentlichungstag und Bekanntmachung des Hinweises auf die Patenterteilung: **02.04.2003**
(21) Anmeldenummer: 97112195.9
(22) Anmeldetag: 17.07.1997
(51) Int. Cl.: A61K 7/13

(54) **Verwendung von Aldehyden zum Färben von keratinhaltigen Fasern**
Use of aldehydes for dyeing keratinic fibres
Utilisation d'aldéhydes pour la coloration des fibres kératiniques

(30) Priorität: 26.07.1996 DE 19630274
(43) Veröffentlichungstag der Anmeldung: 28.01.1998
(73) Patentinhaber: Henkel Kommanditgesellschaft auf Aktien, 40589 Düsseldorf-Holthausen (DE)
(72) Erfinder: Möller, Hinrich, Dr.-Dipl.-Chem., 40789 Monheim (DE); Höffkes, Horst, Dr.-Dipl.-Chem., 40595 Düsseldorf (DE); Meinigke, Bernd, Dr.-Dipl.-Chem., 51399 Burscheid (DE)

(56) Entgegenhaltungen:
- EP-A- 0 370 492
- FR-A- 2 112 549
- US-A- 3 904 357
- US-A- 4 391 603

## Beschreibung

Die Erfindung betrifft die Verwendung von Benz- und Zimtaldehyden zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone und m-Aminophenole verwendet Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 2-Chlor-resorcin, 4-Chlorresorcin, 2-Chlor-6-methyl-3-aminophenol, 2-Methylresorcin und 5-Methylresorcin.

Bezüglich weiterer üblicher Farbstoffkomponenten wird ausdrücklich auf die Colipa-Liste, herausgegeben vom Industrieverband Körperpflege und Waschmittel, Frankfurt, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die deutsche Auslegeschrift DE 28 30 497 offenbart Haarfärbemittel, die Salicylaldehyd in Kombination mit Oxidationsfarbstoffvorprodukten enthalten, z.B. 2,5-Diaminotoluol, 4-Aminophenol oder 2,4-Diaminotoluol, die jedoch (in Kombination mit dem zusätzlich enthaltenen Wasserstoffperoxid) ein mehr oder weniger großes Sensibilisierungspotential aufweisen können. Die deutsche Auslegeschrift DE 29 32 489 offenbart ebenfalls H₂O₂-haltige Haarfärbemittel, die Benzaldehyde, z.B. 2-Hydroxy-3-methoxybenzaldehyd oder 4-Hydroxy-3-methoxybenzaldehyd, in Kombination mit Oxidationsfarbstoffvorprodukten enthalten. Die Patentschriften US 5,034,014 und US 5,199,954 beschreiben Haarfärberezeptur-Beispiele, die p-Dimethylaminobenzaldehyd oder p-Dimethylaminozimtaldehyd, z.B. in Kombination mit dem sensibilisierend wirkenden p-Phenylendiamin enthalten.

Die Patentschrift US 4,391,603 hat oxidationsmittelfreie Haarfärbemittel zum Gegenstand, die substituierte Benzaldehyde enthalten. Die dort offenbarten Haarfärbemittel stellen direktziehende Haarfärbemittel dar, mit denen sich nicht die Farbnuancen, Farbtiefen und Farbechtheiten von Oxidationshaarfarbstoffvorprodukthaltigen Haarfärbemitteln erreichen lassen.

Die Verwendung der unten näher beschriebenen aromatischen Aldehyde zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ mit üblichen Oxidationshaarfärbemitteln mindestens gleichwertig sind, ohne jedoch unbedingt auf Oxidationsmittel wie z.B. H₂O₂ angewiesen zu sein. Darüber hinaus dürfen die Färbemittel kein oder lediglich ein sehr geringes Sensibilisierungspotential aufweisen.

Gegenstand der Erfindung ist die Verwendung von Aldehyden mit der Formel I 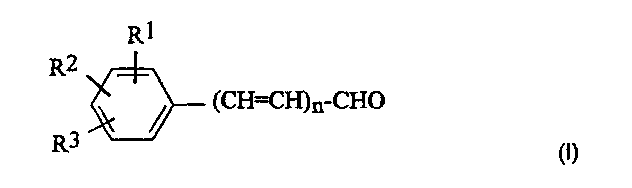 in der R¹ für einen über N mit dem aromatischen Ring verbundenen N-heterocyclischen Rest aus der Gruppe der Pyrrolidine, Piperidine, Morpholine, Piperazidine, Imidazole 1,2,4-, 1,2,3-Triazole oder Azepine steht, R² und R³ gleich oder verschieden sein können und Wasserstoff-, ein Halogenatom, eine Hydroxygruppe, eine C₁₋₄-Alkyl-, Hydroxy-(C₁₋₄)-alkyl- oder C₁₋₄-Alkoxygruppe bedeuten, wobei R² und R³ auch miteinander verbunden sein können, so daß ein ankondensierter isocyclischer oder heterocyclischer, gesättigter oder ungesättigter 5-, 6-oder 7-Ring resultiert, und n für 0 oder 1 steht, zum Färben von keratinhaltigen Fasern, insbesondere menschlichen Haaren.

Überraschenderweise wurde nun gefunden, daß die in der Formel (I) dargestellten Aldehyde sich auch in Abwesenheit von oxidierenden Agentien hervorragend zum Färben von keratinhaltigen Fasern eignen. Sie ergeben Ausfärbungen mit hervorragender Brillianz und Farbtiefe und führen zu vielfältigen Farbnuancen. Der Einsatz von oxidierenden Agentien soll dabei jedoch nicht prinzipiell ausgeschlossen werden.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasem, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrylnitril,-, Polyurethan- und Polyesterfasern verwendet werden.

Die Aldehyde der Formel I sowie die Oxidationsfarbstoffvorprodukte sind vorzugsweise in einer Menge von jeweils 0,03 bis 65, insbesondere 1 bis 40, mmol jeweils bezogen auf 100 g des gesamten Färbemittels, enthalten.

Der Aldehyde der Formel I sind vorzugsweise ausgewählt aus der Gruppe 4-Pyrrolidino-, 4-Piperidino-, 4-Morpholino-, 4-Perhydroazepino-, 4-(1-Imidazolyl)-, 2-Hydroxy-4-pyrrolidino-benzaldehyd, 4-Piperidino-1-naphthaldehyd, 4-Pyrrolidino-, 4-Piperidinozimtaldehyd sowie deren Gemischen.

Diese Substanzen sind i.a. literaturbekannt oder im Handel erhältlich.

Die Aldehyde der Formel (I) alleine färben Keratinfasern im allgemeinen nur im Gelbbereich. Brillante Färbungen im Gelb-, Orange-, Braun- und Schwarzbereich mit noch weiter verbesserten Echtheitseigenschaften (Lichtechtheit, Waschechtheit, Reibechtheit) werden erzielt, wenn die Aldehyde der Formel I gemeinsam mit Verbindungen mit primärer oder sekundärer Aminogruppe, z.B. Anilinderivaten, mit einer Stickstoff enthaltenden heterocyclischen Verbindung, z.B. primären heteroaromatischen Aminen, oder einer aromatischen Hydroxyverbindung verwendet werden. Dies sind einerseits Verbindungen, die für sich alleine keratinhaltige Fasern nur schwach färben und erst gemeinsam mit den Aldehyden der Formel I brillante Färbungen ergeben. Andererseits sind darunter aber auch Verbindungen, die bereits als Oxidationsfarbstoffvorprodukte eingesetzt werden.

Unter diese Ausführungsform fällt auch die Verwendung solcher Substanzen, die Reaktionsprodukte von Aldehyden der Formel I mit den genannten Verbindungen darstellen.

Geeignete Verbindungen mit primärer oder sekundärer Aminogruppe sind z.B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, o-, m-, p-Phenylendiamin, o-, m-, p-Toluylendiamin, 2,5-Diaminotoluol, -phenol, -anisol, -phenethol, 4-Amino-3-methylphenol, 2-(2,5-Diaminophenyl)-ethanol, 2-(2,5-Diaminophenoxy)-ethanol, 2-Chlor-p-phenylendiamin, 4-Methylamino-, 3-, 4-Dimethylamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-6-chlor-, 2-Methyl-5-amino-4-chlor-, 2-Methyl-5-amino-6-chlor-, 5-(2-Hydroxyethylamino)-4-methoxy-2-methyl-, 4-Amino-2-aminomethyl-phenol, 1,3-Diamino-2,4-dimethoxybenzol, 1-(β-Hydroxyethyl)-2,5-diaminobenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest , wie sie in der Formel II dargestellt sind 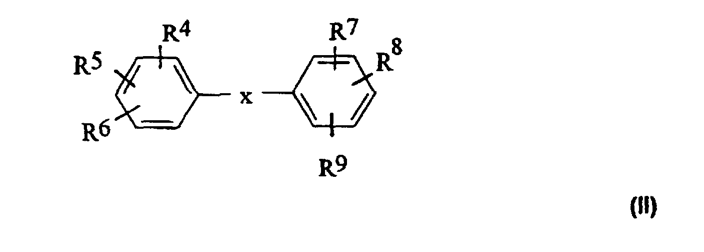 in der R⁴ für eine Hydroxy- oder eine Aminogruppe, die durch C₁₋₄-Alkyl, C₁₋₄-Hydroxyalkyl, C₁₋₄-Alkoxy- oder C₁₋₄-Alkoxy-C₁₋₄-alkyl substituiert sein kann, stehen, R⁵, R⁶, R⁷, R⁸ und R⁹ für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋-₄-alkylgruppe substituiert sein können, stehen, und
X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

Z-(CH₂-Y-CH₂-Z')ₒ (III)

in der Y eine direkte Bindung, eine CH₂- oder CH₂OH-Gruppe,
Z und Z' für ein Sauerstoffatom oder eine NR¹⁰, worin R¹⁰ Wasserstoff, eine C₁₋-₄-Alkyl-, Hydroxy-C₁₋₄-alkylgruppe, die Gruppe -O-CH₂(CH₂)ₚ-NH oder NH-CH₂(CH₂)_{p'}, worin p und p' 0 oder 1 sind, und
o eine Zahl von 1 bis 4 bedeuten,
steht, wie beispielsweise 4,4'-Diaminostilben, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin, N-Phenyl-1,4-phenylendiamin. Die vorgenannten Verbindungen können sowohl in freier Form als auch in Form ihrer physiologisch verträglichen Salze, z. B. als Salze anorganischer Säuren, wie Salzoder Schwefelsäure, eingesetzt werden.

Geeignete stickstoffhaltige heterocyclische Verbindungen sind z.B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 4-, 5-, 6-, 7-Aminoindol, 5-, 6-Aminoindazol, 5-, 7-Aminobenzimidazol, -benzothiazol, 2,5-Dihydroxy-4-morpholinoanilin sowie Indol- und Indolinderivaten, wie 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salzoder Schwefelsäure, eingesetzt werden.

Geeignete aromatische Hydroxyverbindungen sind z.B. 2-, 4-, 5-Methylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Methyl-, 2-, 4-Chlorresorcin, 1-, 2-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als Aminosäuren kommen alle natürlich vorkommenden und synthetischen Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenyldialanin), Ornithin, Lysin und Tryptophan.

Die Oligopeptide können dabei natürlich vorkommende oder synthetische Oligopeptide, aber auch die in Polypeptid- oder Proteinhydrolysaten enthaltenen Oligopeptide sein, sofem sie über eine für die Anwendung in den erfindungsgemäßen Färbemitteln ausreichende Wasserlöslichkeit verfügen. Als Beispiele sind z.B. Glutathion und die in den Hydrolysaten von Kollagen, Keratin, Casein, Elastin, Sojaprotein, Weizengluten oder Mandelprotein enthaltenen Oligopeptide zu nennen.

Bevorzugt ist dabei die Verwendung gemeinsam mit Verbindungen mit primärer oder sekundärer Aminogruppe oder mit aromatischen Hydroxyverbindungen.

In allen Färbemitteln können auch mehrere verschiedene Aldehyde der Formel I gemeinsam zum Einsatz kommen; ebenso können auch mehrere verschiedene Komponenten aus den Gruppen von Verbindungen mit primärer oder sekundärer Aminogruppe, von stickstoffhaltigen Heterocyclen, aromatischen Hydroxyverbindungen oder Aminosäuren gemeinsam verwendet werden.

Auf die Anwesenheit von Oxidationsmitteln, z.B. H₂O₂, kann dabei verzichtet werden. Es kann jedoch u.U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt Ein für menschliches Haar bevorzugtes Oxidationsmittel ist H₂O₂.

Die erfindungsgemäßen Färbemittel ergeben eine breite Palette von Farbnuancen im Bereich von gelborange bis braunschwarz; die Echtheitseigenschaften sind hervorragend, die Sensibilisierungspotentiale sehr gering.

In einer bevorzugten Ausführungsform enthalten die erfindungsgemäßen Färbemittel zur weiteren Modifizierung der Farbnuancen neben den Oxidationsfarbstoffvorprodukten zusätzlich übliche direktziehende Farbstoffe, z.B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16, Pikraminsäure und Rodol 9 R bekannten Verbindungen. sowie 4-N-Ethyl-1,4-bis-(2'-hydroxyethyl-amino)-2-nitrobenzol, 1-(2'-Hydroxyethyl)-amino-4-methyl-2-nitro-benzol, 1-Amino-2-nitro-4-(2'hydroxyethylamino)-5-chlorbenzol, 4-Amino-2-nitro-diphenylamin-2'-carbonsäure und 6-Nitro-1,2,3,4-tetrahydrochinoxalin in einer Menge von 0,01 bis 20 Gew.-%, bezogen auf das gesamte Oxidationshaarfärbemittel.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- undloder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller Dow Coming; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex® vertriebenen Dialkylammoniummethosulfate und Methyl-hydroxyalkyldialkoyloxyalkyl-ammoniummethosulfate.

Ein Beispiel für ein als kationisches Tensid einsetzbares quatemäres Zuckerderivat stellt das Handelsprodukt Glucquat® 100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthän-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellutose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Hamstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Zum Färben der keratinhaltigen Fasern, insbesondere zum Färben von menschlichen Haaren, werden die Färbemittel in der Regel in Form des wasserhaltigen, kosmetischen Trägers in einer Menge von 100 g auf das Haar aufgebracht, ca. 30 Minuten dort belassen und anschließend ausgespült oder mit einem handelsüblichen Haarshampoo ausgewaschen.

Die Aldehyde der Formel I und die Oxidationsfarbstoffvorprodukte können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die Aldehyde der Formel I und die Oxidationsfarbstoffvorprodukte können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (50 bis 80°C) Wassers hinzugefügt und eine homogene Mischung hergestellt.

### Beispiele

### Herstellung einer Färbelösung

Es wurde eine Aufschlämmung von 10 mMol eines Aldehyds der Formel I, 10 mMol eines Oxidationsfarbstoffvorproduktes, 10 mMol Natriumacetat und ein Tropfen einer 20 %igen Fettalkylethersulfat-Lösung in 100 ml Wasser bereitet. Die Aufschlämmung wurde kurz auf ca. 80°C erhitzt und nach dem Abkühlen filtriert, der pH-Wert wurde anschließend auf 6 eingestellt.

In diese Färbelösung wurde bei 30°C 30 Minuten lang eine Strähne zu 90% ergrauten, nicht vorbehandelten Menschenhaares eingebracht Die Strähne wurde dann 30 Sek. mit lauwarmen Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:
- - :: keine oder eine sehr blasse Ausfärbung
- (+):: schwache Intensität
- +:: mittlere Intensität
- +(+) :: mittlere bis starke Intensität
- ++:: starke Intensität
- ++(+) :: starke bis sehr starke Intensität
- +++ :: sehr starke Intensität

**Tabelle1**

| **Ausfärbungen mit 4-Pyrrolidinobenzaldehyd** | |
|---|---|
| Oxidationsfarbstoffvorprodukt | Färbenuance |
| | Farbtiefe |
| 2,5-Diaminotoluol x H₂SO₄ ++(+) | rotbraun |
| 2,4,5,6-Tetraaminopyrimidin x H₂SO₄ +(+) | orangegelb |
| 2-Methylamino-3-amino-6-methoxypyridin x 2HCl ++(+) | dunkelbraun |
| 2-(2,5-Diaminophenyl)-ethanol x H₂SO₄ ++(+) | rotorange |
| 2-Aminomethyl-4-aminophenol x 2HCl ++(+) | goldgelb |
| N,N-Bis-(2-hydroxyethyl)-p-phenylendiamin x H₂SO₄ | rot +++ |
| 4,4'-Diaminodiphenylamin x H₂SO₄ | braunviolett +++ |
| 2,6-Dimethoxy-3,5-diaminopyridin x 2HCl +++ | braunschwarz |

## Patentansprüche

1. Verwendung von Aldehyden mit der Formel I 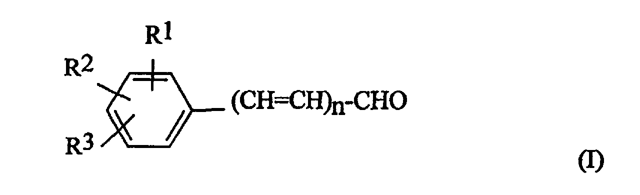 in der R¹ für einen über N mit dem aromatischen Ring verbundenen N-heterocyclischen Rest aus der Gruppe der Pyrrolidine, Piperidine, Morpholine, Piperazidine, Imidazol, 1,2,4-, 1,2,3-Triazole oder Azepine steht, R² und R³ gleich oder verschieden sein können und Wasserstoff-, ein Halogenatom, eine Hydroxygruppe, eine C₁₋₄-Alkyl-, Hydroxy-(C₁₋₄)-alkyl- oder C₁₋₄-Alkoxygruppe bedeuten, wobei R² und R³ auch miteinander verbunden sein können, so daß ein ankondensierter isocyclischer oder heterocyclischer, gesättigter oder ungesättigter 5-, 6- oder 7-Ring resultiert, und n für 0 oder 1 steht, zum Färben von keratinhaltigen Fasern, insbesondere menschlichen Haaren.

2. Verwendung nach Anspruch 1, **dadurch gekennzeichnet, daß** die Aldehyde der Formel I in einer Menge von jeweils 0,03 bis 65, insbesondere 1 bis 40, mmol jeweils bezogen auf 100 g des gesamten Färbemittels, enthalten sind.

3. Verwendung nach Anspruch 1 oder 2, **dadurch gekennzeichnet, daß** der Aldehyd der Formel (I) ausgewählt ist aus 4-Pyrrolidino-, 4-Piperidino-, 4-Morpholino-, 4-Perhydroazepino-, 4-(1-Imidazolyl)-, 2-Hydroxy-4-pyrrolidinobenzaldehyd sowie deren Gemischen.

4. Verwendung nach einem der Ansprüche 1 bis 3, **dadurch gekennzeichnet, daß** zusätzlich mindestens eine weitere Verbindungen mit primärer oder sekundärer Aminogruppe und/oder einer Stickstoff enthaltenen heterocyclischen Verbindung und/oder eine aromatische Hydroxyverbindung eingesetzt wird.

5. Verwendung nach Anspruch 4, **dadurch gekennzeichnet, daß** die Verbindungen mit primärer oder sekundärer Aminogruppe ausgewählt sind aus primären aromatischen Aminen wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-pphenylendiamin, 2-Chlor-p-phenylendiamin, 2,5-Dihydroxy-4-morpholinoanilindihydrobromid, 2-, 3-, 4-Aminophenol, 2-Aminomethyl-4-aminophenol, o-, m-, p-Phenylendiamin, o-, m-, p-Toluylendiamin, 2,5-Diaminotoluol, -phenol, -anisol, -phenethol, 4-Amino-3-methylphenol, 2-(2,5-Diaminophenyl)-ethanol, 2-(2,5-Diaminophenoxy)-ethanol, 2-Chlor-p-phenylendiamin, 4-Methylamino-, 3-, 4-Dimethylamino-, 3,4-Methylendioxyanilin, 3-Amino-2,4-dichlor-, 4-Methylamino-, 2-Methyl-5-amino 3-Methy-4-amino-, 2-Methyl-5-(2-hydroxyethylamino)-, 2-Methyl-5-amino-6-chlor-, 2-Methyl-5-amino-4-chlor-, 2-Methyl-5-amino-6-chlor-, 5-(2-Hydroxyethylamino)-4-methoxy-2-methyl-, 4-Amino-2-aminomethyl-phenol, 1,3-Diamino-2,4-dimethoxybenzol, 1-(β-Hydroxyethyl)-2,5-diaminobenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest, wie sie in der Formel II dargestellt sind 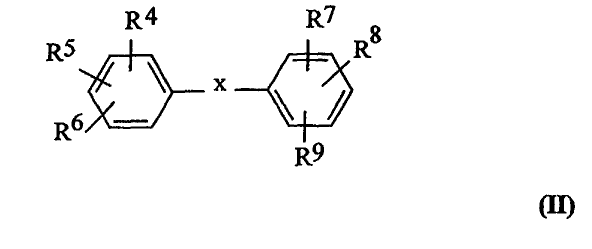 in der
• R⁴ für eine Hydroxy- oder eine Aminogruppe, die durch C₁₋₄-Alkyl, C₁₋₄-Hydroxyalkyl, C₁₋₄-Alkoxy- oder C₁₋₄-Alkoxy-C₁₋₄-alkyl substituiert sein kann, stehen,
• R⁵, R⁶, R⁷, R⁸ und R⁹ für Wasserstoff, eine Hydroxy- oder eine Aminogruppe, die durch C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppe substituiert sein können, stehen, und
• X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III
Z-(CH₂-Y-CH₂-Z')ₒ (III)
in der
• Y eine direkte Bindung, eine CH₂- oder CH₂OH-Gruppe,
• Z und Z' für ein Sauerstoffatom oder eine NR¹⁰, worin R¹⁰ Wasserstoff, eine C₁₋₄-Alkyl-, Hydroxy-C₁₋₄-alkylgruppe, die Gruppe -O-CH₂(CH₂)ₚ-NH oder NH-CH₂(CH₂)_{p'}, worin p und p' 0 oder 1 sind, und
• o eine Zahl von 1 bis 4 bedeuten,
steht,
wie 4,4'-Diaminostilben, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]methylamin und N-Phenyl-1,4-phenylendiamin, sowie den physiologisch verträglichen Salzen aller vorgenannten Verbindungen.

6. Verwendung nach Anspruch 4, **dadurch gekennzeichnet, daß** die stickstoffhaltigen heterocyclischen Verbindungen ausgewählt sind aus 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4 methoxy-6-methyl-pyrimidin, 3,5-Diaminopyrazol, -1,2,4-triazol, 3-Amino-, 3-Amino-5-hydroxypyrazol, 2-,3-, 8-Aminochinolin, 4-Amino-chinaldin, 2-, 6-Aminonicotinsäure, 5-Aminoisochinolin, 4-, 5-, 6-, 7-Aminoindol, 5-, 6-Aminoindazol, 5-, 7-Aminobenzimidazol, -benzothiazol, 2,5-Dihydroxy-4-morpholinoanilin sowie Indol- und Indolinderivaten, wie 5,6-Dihydroxyindol, 5,6-Dihydroxyindolin und 4-Hydroxyindolin, sowie deren physiologisch verträglichen Salzen.

7. Verwendung nach Anspruch 4, **dadurch gekennzeichnet, daß** die aromatischen Hydroxyverbindungen ausgewählt sind aus 2-, 4-, 5-Methylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure,phenylessigsäure, Gallussäure, 2,4,6-Trihydroxybenzoesäure, -acetophenon, 2-, 4-Methyl-, 2-, 4-Chlorresorcin, 1-, 2-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

8. Verwendung nach einem der Ansprüche 1 bis 7, **dadurch gekennzeichnet, daß** Zusätzlich Oxidationsmittel in einer Menge von 0,01 bis 6 Gew.-%, bezogen auf die Anwendungslösung, eingesetzt werden.

9. Verwendung nach Anspruch 8, **dadurch gekennzeichnet, daß** als Oxidationsmittel H₂O₂ eingesetzt wird.

10. Verwendung nach einem der Ansprüche 1 bis 9, **dadurch gekennzeichnet, daß** anionische, zwitterionische und/oder nichtionische Tenside eingesetzt werden.

## Claims

1. The use of aldehydes corresponding to formula I: 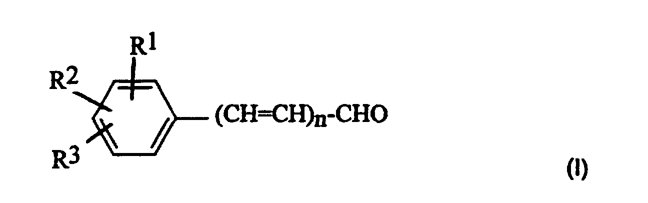 in which R¹ is an N-heterocyclic radical attached by N to the aromatic ring from the group of pyrrolidines, piperidines, morpholines, piperazidines, imidazole, 1,2,4-, 1,2,3-triazoles or azepines, R² and R³ may be the same or different, represent hydrogen, a halogen atom, a hydroxy group, a C₁₋₄ alkyl, hydroxy-(C₁₋₄)-alkyl or C₁₋₄ alkoxy group and may even be joined together so that a fused isocyclic or heterocyclic, saturated or unsaturated 5-, 6- or 7-membered ring is formed, and n = 0 or 1, for coloring keratin-containing fibers, more especially human hair.

2. The use claimed in claim 1, **characterized in that** the aldehydes corresponding to formula I are present in a quantity of 0.03 to 65 and more more particularly 1 to 40 mmol per 100 g of the colorant as a whole.

3. The use claimed in claim 1, **characterized in that** the aldehyde of formula (I) is selected from 4-pyrrolidino, 4-piperidino, 4-morpholino, 4-perhydroazepino, 4-(1-imidazolyl)-, 2-hydroxy-4-pyrrolidino-benzaldehyde and mixtures thereof.

4. The use claimed in any of claims 1 to 3, **characterized in that** at least one other compound containing a primary or secondary amino group and/or a nitrogen-containing heterocyclic compound and/or an aromatic hydroxy compound is additionally used.

5. The use claimed in claim 4, **characterized in that** the compounds containing a primary or secondary amino group are selected from primary aromatic amines, such as N,N-dimethyl-, N,N-diethyl-, N-(2-hydroxyethyl)-N-ethyl-, N,N-bis-(2-hydroxyethyl)-, N-(2-methoxyethyl)-, 2,3-, 2,4- and 2,5-dichloro-p-phenylenediamine, 2-chloro-p-phenylenediamine, 2,5-dihydroxy-4-morpholinoaniline dihydrobromide, 2-, 3-, 4-aminophenol, 2-aminomethyl-4-aminophenol, o-, m-, p-phenylenediamine, o-, m-, p-toluylenediamine, 2,5-diaminotoluene, -phenol, -anisole and -phenethol, 4-amino-3-methylphenol, 2-(2,5-diaminophenyl)-ethanol, 2-(2,5-diaminophenoxy)-ethanol, 2-chloro-p-phenylenediamine, 4-methylamino-, 3-, 4-dimethylamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-,2-methyl-5-amino-6-chloro-, 5-(2-hydroxyethylamino)-4-methoxy-2-methylphenol, 4-amino-2-aminomethylphenol, 1,3-diamino-2,4-dimethoxybenzene, 1-(β-hydroxyethyl)-2,5-diaminobenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulfonic acid, 3-amino-4-hydroxybenzenesulfonic acid, 4-amino-3-hydroxynaphthalene-1-sulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechol, 4,6-diaminopyrogallol, 3,5-diamino-4-hydroxypyrocatechol, aromatic anilines and phenols containing another aromatic radical corresponding to formula (II): 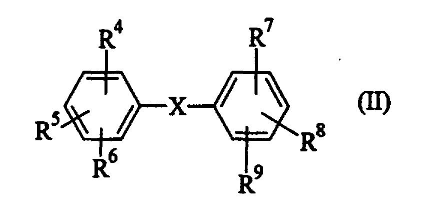 in which
• R⁴ is a hydroxy group or an amino group which may be substituted by C₁₋₄ alkyl, C₁₋₄ hydroxyalkyl, C₁₋₄-alkoxy- or C₁₋₄-alkoxy-C₁₋₄-alkyl groups,
• R⁵, R⁶, R⁷, R⁸ and R⁹ represent hydrogen, a hydroxy group or an amino group, which may be substituted by C₁₋₄ alkyl, C₁₋₄ hydroxyalkyl, C₁₋₄-alkoxy-, C₁₋₄ aminoalkyl or C₁₋₄-alkoxy-C₁₋₄-alkyl groups and
• X is a direct bond, a saturated or unsaturated, optionally hydroxy-substituted carbon chain containing 1 to 4 carbon atoms, a carbonyl, sulfonyl or imino group, an oxygen or sulfur atom or a group corresponding to formula (III):
Z-(CH₂-Y-CH₂-Z')ₒ (III)
in which
• Y is a direct bond, a CH₂ or CHOH group,
• Z and Z' independently of one another represent an oxygen atom or an NR¹⁰ group, where R¹⁰ is hydrogen, a C₁₋₄ alkyl or a hydroxy-C₁₋₄-alkyl group, the group O-(CH₂)ₚ-NH or NH-CH₂(CH₂)_{p'}, where p and p' = 0 or 1, and
• o is a number of 1 to 4,
such as for example 4,4'-diaminostilbene, 4,4'-diaminostilbene-2,2'-disulfonic acid, Na salt, 4,4'-diaminodiphenyl methane, -sulfide, -sulfoxide, -amine, 4,4'-diaminodiphenylamine-2-sulfonic acid, 4,4'-diaminobenzophenone, -diphenyl ether, 3,3',4,4'-tetraaminodiphenyl, 3,3'4,4'-tetraaminobenzophenone, 1,3-bis-(2,4-diaminophenoxy)-propane, 1,8-brs-(2,5-diaminophenoxy)-3,6-dioxaoctane, 1,3-bis-(4-aminophenylamino)-propane, -2-propanol, 1,3-bis-[N-(4-aminophenyl)-2-hydroxyethylamino]-2-propanol, N,N-bis-[2-(4-aminophenoxy)-ethyl]-methylamine and N-phenyl-1,4-phenylenediamine, and physiologically compatible salts of the above-mentioned compounds.

6. The use claimed in claim 4, **characterized in that** the nitrogen-containing heterocyclic compounds are selected from 2-, 3-, 4-amino-, 2-amino-3-hydroxy-, 2,6-diamino-, 2,5-diamino-, 2,3-diamino-, 2-dimethylamino-5-amino-, 2-methylamino-3-amino-6-methoxy-, 2,3-diamino-6-methoxy-, 2,6-dimethoxy-3,5-diamino-, 2,4,5-triamino-, 2,6-dihydroxy-3,4-dimethyl pyridine, 2,4-dihydroxy-5,6-diamino-, 4,5,6-triamino-, 4-hydroxy-2,5,6-triamino-, 2-hydroxy-4,5,6-triamino-, 2,4,5,6-tetraamino-, 2-methylamino-4,5,6-triamino-, 2,4-, 4,5-diamino-, 2-amino-4-methoxy-6-methyl pyrimidine, 3,5-diaminopyrazole, -1,2,4-triazole, 3-amino-, 3-amino-5-hydroxypyrazole, 2-, 3-, 8-aminoquinoline, 4-aminoquinaldine, 2-, 6-aminonicotinic acid, 5-aminoisoquinoline, 4-, 5-, 6-, 7-aminoindole, 5-, 6-aminoindazole, 5-, 7-aminobenzimidazole, -benzothiazole, 2,5-dihydroxy-4-morpholinoaniline and indole and indoline derivatives, such as 5,6-dihydroxyindole, 5,6-dihydroxyindoline and 4-hydroxyindoline and physiologically compatible salts thereof.

7. The use claimed in claim 4, **characterized in that** the aromatic hydroxy compounds are selected from 2-, 4-, 5-methylresorcinol, resorcinol, 3-methoxyphenol, pyrocatechol, hydroquinone, pyrogallol, phloroglucinol, hydroxyhydroquinone, 2-, 3-, 4-methoxy-, 3-dimethylamino, 2-(2-hydroxyethyl)-, 3,4-methylenedioxyphenol, 2,4-, 3,4-dihydroxybenzoic or phenylacetic acid, gallic acid, 2,4,6-trihydroxybenzoic acid, -acetophenone, 2-, 4-methyl-, 2-, 4-chlororesorinol, 1-, 2-naphthol, 1,5-, 2,3-, 2,7-dihydroxynaphthalene, 6-dimethylamino-4-hydroxy-2-naphthalenesulfonic acid, 3,6-dihydroxy-2,7-naphthalenedisulfonic acid.

8. The use claimed in any of claims 1 to 7, **characterized in that** oxidizing agents are additionally used in a quantity of 0.01 to 6% by weight, based on the solution used.

9. The use claimed in claim 8, **characterized in that** H₂O₂ is used as the oxidizing agent.

10. The use claimed in any of claims 1 to 9, **characterized in that** anionic, zwitterionic and/or nonionic surfactants are used.

## Revendications

1. Utilisation d'aldéhydes de la formule I 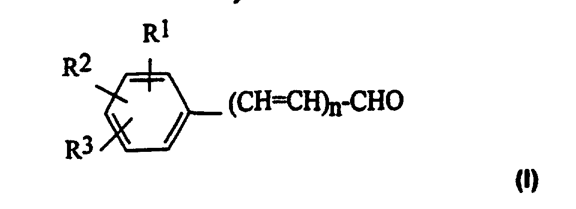 dans laquelle R¹ représente un radical N-hétérocyclique, lié au cycle aromatique via N, appartenant au groupe des pyrrolidines, pipéridines, morpholines, pipérazidines, imidazole, 1,2,4-, 1,2,3-triazoles ou azépines, R² et R³ peuvent être identiques ou différents et correspondre à un atome d'hydrogène, un atome d'halogène, un groupe hydroxyle, un groupe alkyl en C₁₋₄ , hydroxy-alkyl en C₁₋₄ ou alcoxy en C₁₋₄ , R² et R³ pouvant également être liés entre eux, de manière à former un noyau à 5, 6 ou 7 membres accolé isocyclique ou hétérocyclique, saturé ou insaturé, et n vaut 0 ou 1, pour colorer des fibres kératiniques, en particulier des cheveux ou des poils humains.

2. Utilisation selon la revendication 1, **caractérisée en ce que** les aldéhydes de la formule I sont contenus chaque fois dans une proportion de 0,03 à 65, en particulier de 1 à 40 mmol, dans chaque cas pour 100 g de la totalité du produit colorant.

3. Utilisation selon la revendication 1 ou 2, **caractérisée en ce que** l'aldéhyde de la formule I est sélectionné parmi le 4-pyrrolidino-, le 4-pipéridino-, le 4-morpholino, le 4-perhydroazépino-, le 4-(1-imidazolyl)- et le 2-hydroxy-4-pyrrolidinobenzaldéhyde et les mélanges de ceux-ci.

4. Utilisation selon une des revendications 1 à 3, **caractérisée en ce qu'**on met en oevre au moins un autre composé comportant un groupe amino primaire ou secondaire et/ou un composé hétérocyclique azoté et/ou un composé hydroxylé aromatique.

5. Utilisation selon la revendication 4, **caractérisée en ce que** les composés comportant un groupe amino primaire ou secondaire sont sélectionnés parmi les amines aromatiques primaires telles que les composés suivants: N,N-diméthyl-, N,N-diéthyl-, N-(2-hydroxyéthyl)-N-éthyl, N,N-bis-(2-hydroxyéthyl)-, N-(2-méthoxyéthyl-), 2,3- 2,4-, 2,5-dichloro-p-phénylènediamine, 2-chloro-p-phénylènediamine, 2,5-dihydroxy-4-morpholinoanilino-dihydrobromure, 2-, 3-, 4-aminophénol, 2-aminométhyl-4-aminophénol, o-, m-, p-phénylènediamine, o-, m-, p-toluylènediamine, 2,5-diaminotoluène, -phénol, -anisol, -phénéthol, 4-amino-3-méthylphénol, 2-(2,5-diaminophényl)-éthanol, 2-(2,5-diaminophénoxy)-éthanol, 2-chloro-p-phénylènediamine, 4-méthylamino-, 3-, 4- diméthylamino-, 3,4-méthylènedioxyaniline, 3-amino-2,4-dichloro-, 4-méthylamino-, 2-méthyl-5-amino-, 3-méthyl-4-amino, 2-méthyl-5-(2-hydroxyéthylamino)-, 2-méthyl-5-amino-6-chloro-, 2-méthyl-5-amino-4-chloro-, 2-méthyl-5-amino-6-chloro-, 5-(2-hydroxyéthylamino)-4-méthoxy-2-méthyl-, 4-amino-2-aminométhyl-phénol, 1,3-diamino-2,4-diméthoxybenzène, 1-(β-hydroxyéthyl)2,5-diaminobenzène, acide 2-, 3-, 4-aminobenzoïque, acide 2-, 3-, 4-phénylacétique, acide 2,3- 2,4-, 2,5- 3,4-, 3,5-diaminobenzoïque, acide 4-, 5-aminosalicylique, acide 3-amino-4-hydroxy-, 4-amino-4-hydroxybenzoïque, acide 2-, 3-, 4-aminobenzènesulfonique, acide 3-amino-4-hydroxybenzènesulfonique, acide 4-amino-3-hydroxynaphtalène-1-sulfonique, acide 6-amino-7-hydroxynaphtalène-2-sulfonique, acide 7-amino-4-hydroxynaphtaléne-2-sulfonique, acide 4-amino-5-hydroxynaphtalène-2,7-disulfonique, acide 3-amino-2-naphtoïque, acide 3-aminophtalique, acide 5-aminoisophtalique, 1,3,5-, 1,2,4-triaminobenzène, 1,2,4,5-tétraaminobenzène, 2,4,5-triaminophénol, pentaaminobenzène, hexaaminobenzène, 2,4,6-triaminorésorcine, 4,5-diaminopyrocatéchine, 4,6-diaminopyrogallol, 3,5-diamino-4-hydroxypyrocatéchine, anilines ou phénols aromatiques comportant un autre radical aromatique, tels que ceux qui sont représentés dans la formule II 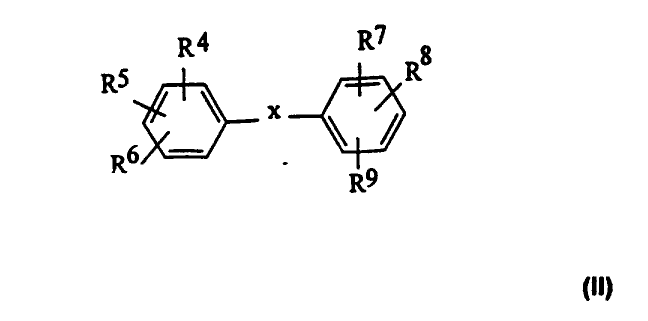 dans laquelle
• R⁴ représente un groupe hydroxyle ou un groupe amino, qui peut être substitué par un groupe alkyl en C₁₋₄, hydroxyalkyl en C₁₋₄, alcoxy en C₁₋₄ ou alcoxy en C₁₋₄ -alkyl en C₁₋₄,
• R⁵, R⁶, R⁷, R⁸ et R⁹ correspondent à l'hydrogène, à un groupe hydroxyle ou à un groupe amino, qui peuvent être substitués par un groupe alkyl en C₁₋₄, hydroxyalkyl en C₁₋₄, alcoxy en C₁₋₄, aminoalkyl en C₁₋₄ ou alcoxy en C₁₋₄ -alkyl en C₁₋₄,
• X est une liaison directe, une chaîne hydrocarbonée comportant 1 à 4 atomes de carbone, saturée ou insaturée, éventuellement substituée par des groupes hydroxyle, un groupe carbonyle, sulfonyle ou imino, un atome d'oxygène ou un atome de soufre, ou un groupe de formule III
Z-(CH₂-Y-CH₂-Z')ₒ (III)
dans laquelle
• Y est une liaison directe, un groupe CH₂ ou un groupe CH₂OH,
• Z et Z' représentent un atome d'oxygène ou un groupe NR¹⁰, dans lequel R¹⁰ représente l'hydrogène, un groupe alkyl enC₁₋₄, hydroxyalkyl en C₁₋₄ , le groupe -O-CH₂(CH₂)ₚ-NH ou le groupé NH-CH₂(CH₂)_{p'},
dans lequel p et p' valent 0 ou 1, et
• o est un nombre de 1 à 4,
tels que les composés suivants: 4,4'-diaminostilbène, acide 4,4'-diaminostilbène-2,2'-disulfonique, sel sodique, 4,4'-diaminodiphénylméthane, -sulfure, -sulfoxyde, -amine, acide 4,4'diaminodiphénylamine-2-sulfonique, 4,4'-diaminobenzophénone, -diphényléther, 3,3',4,4'-tétraaminodiphényl, 3,3',4,4'-tétraamino-benzophénone, 1,3-bis-(2,4-diaminophénoxy)-propane, 1,8-bis-(2,5-diaminophénoxy)-3,6-dioxaoctane, 1,3-bis-(4-aminophénylamino)-propane, -2-propanol, 1,3-bis-[N-(4-aminophényl)-2-hydroxyéthylamino]-2-propanol, N,N-bis-[2-(4-aminophénoxy)-éthyl]méthylamine et N-phényl-1,4-phénylènediamine ainsi que les sels physiologiquement tolérables de tous les composés précités.

6. Utilisation selon la revendication 4, **caractérisée en ce que** les composés hétérocycliques azotés sont sélectionnés parmi les composés suivants: 2-, 3-, 4-amino-, 2-amino-3-hydroxy-, 2,6-diamino-, 2,5-diamino-, 2,3-diamino-, 2-diméthylamino-5-amino-, 2-méthylamino-3-amino-6-méthoxy-, 2,3-diamino-6-méthoxy-, 2,6-diméthoxy-3,5-diamino-, 2,4,5-triamino-, 2,6-dihydroxy-3,4-diméthylpyridine, 2,4-dihydroxy-5,6-diamino-, 4,5,6-triamino, 4-hydroxy-2,5,6-triamino-, 2-hydroxy-4,5,6-triamino-, 2,4,5,6-tétraamino-, 2-méthylamino-4,5,6-triamino-, 2,4-, 4,5-diamino-, 2-amino-4-méthoxy-6-méthyl-pyrimidine, 3,5-diaminopyrazole, -1,2,4-triazole, 3'-amino-, 3-amino-5-hydroxypyrazole, 2-, 3-, 8-aminoquinoléine, 4-amino-quinaldine, acide 2-, 6-aminonicotinique, 5-aminoisoquinoléine, 4-, 5-, 6-, 7-aminoindole, 5-, 6-aminoindazole, 5-, 7-aminobenzimidazole, -benzothiazole, 2,5-dihydroxy-4-morpholinoaniline et dérivés d'indole et d'indoline, tels que le 5-6-dihydroxyindole, la 5,6-dihydroxyindoline et la 4-hydroxyindoline, ainsi que les sels physiologiquement tolérables de ceux-ci.

7. Utilisation selon la revendication 4, **caractérisée en ce que** les composés hydroxyle aromatiques sont sélectionnés parmi les composés suivants: 2-, 4-, 5-méthylrésorcine, résorcine, 3-méthoxyphénol, pyrocatéchine, hydroquinone, pyrogallol, phloroglucine, hydroxyhydroquinone, 2-, 3-, 4-méthoxy-, 3-diméthylamino-, 2-(2-hydroxyéthyl)-, 3,4-méthylènedioxyphénol, acide 2,4-, 3,4-dihydroxybenzoïque, acide 2,4-, 3,4-phénylacétique, acide gallique, acide 2,4,6-trihydroxybenzoïque, 2,4,6-acétophénone, 2-, 4-méthyl-, 2-, 4-chlororésorcine, 1-, 2-naphtol, 1,5-, 2,3-, 2,7-dihydroxynaphtalène, acide 6-diméthylamino-4-hydroxy-2-naphtalènique et acide 3,6-dihydroxy-2,7-naphtalènique.

8. Utilisation selon une des revendications 1 à 7, **caractérisée en ce que** des oxydants sont mis en oeuvre en plus dans une proportion de 0,01 à 6 % en poids par rapport à la solution d'utilisation.

9. Utilisation selon la revendication 8, **caractérisée en ce que** l'on utilise de l'eau oxygénée comme oxydant.

10. Utilisation selon une des revendications 1 à 9, **caractérisée en ce que** l'on met en oeuvre des tensioactifs anioniques, zwitterioniques et/ou non ioniques.
